# EUROPEAN PATENT APPLICATION

(11) **EP 2 100 826 A1**
(43) Date of publication of application: **16.09.2009**
(21) Application number: 07711062.5
(22) Date of filing: 08.03.2007
(51) Int. Cl.: B65F 1/00, C02F 1/50, C02F 1/28, B65D 30/02, B65D 77/06

(54) **MEDICAL SEWAGE TREATER**

(30) Priority: 06.12.2006 CN 200610124036
(71) Applicant: Liu, Xiao, Yuexiu District Guangzhou Guangdong 510-040 (CN); Zhou, Taili, Guangdong 510060 (CN)
(72) Inventor: Liu, Xiao, Yuexiu District Guangzhou Guangdong 510-040 (CN); Zhou, Taili, Guangdong 510060 (CN)
(74) Representative: Papa, Elisabetta
(86) International application number: PCT/CN2007/000741
(87) International publication number: WO 2008/067694

(57) **Abstract**

This invention relates to a medical sewage treater, which consists of an article storing bag and water absorbing material, wherein the water absorbing material is a super absorbent polymer and is adhered to the inner wall of the article storing bag or set in the article storing bag. After medical sewage has flowed into the article storing bag, the water absorbing material absorbs water and expands so as to make the treated medical sewage become a colloid or solid of poor flowability such that it is convenient and safe in transportation and can effectively overcome the disadvantages in the prior art, such as uneasily stacking, leakage and thereby pollution during the transportation of the medical sewage. The invention is also applicable to collection and treatment of other deleterious sewage in daily life.

## Description

### TECHNICAL FIELD

This invention relates to a sewage treater, especially a medical sewage treater.

### BACKGROUND ART

In the art, the medical sewage such as blood, urine, mucous and other body discharge commonly is sucked into plastic bags with a negative pressure source during the operation in hospital, and then carries them to a medical waste disposal center to dispose. Because Medical sewage is fluids and prone to leaking, so must be careful for transportation, and prevent pollution led by the leakage of medical sewage. Deleterious sewage in daily life also needs to be transported easily and safely, but there is not a simple, effective disposal method in the existing technology.

### SUMMARY OF THE INVENTION

The invention intends to develop a medical sewage treater, which can make sewage, especially medical sewage into a colloid or solid of poor flowability for the convenience of transportation.

According to the present invention, there is provided an assembly comprising an article storing bag and water absorbing material, wherein said water absorbing material is material which could make the treated medical sewage become a colloid or solid of poor flowability, and said water absorbing material is adhered to the inner wall of said article storing bag or set in the article storing bag.

According to the further feature of the present invention, the water absorbing material is super absorbent polymer (SAP).

In particularly, the said super absorbent polymer is synthetic polymer series super absorbent polymer.

The super absorbent polymer is selected from a group of starch series, cellulose series or synthetic super absorbent polymer.

The super absorbent polymer is polycyclic acid series super absorbent polymer.

In particularly, the water absorbing material is in the form of powder or particle.

According to the further feature of the present invention, the water absorbing material contains bactericidal reagents or bactericidal materials.

According to the further feature of the present invention, the medical sewage treater further comprising a seal cover, connected to the mouth of the article storing bag; a water inlet pipe; a vacuum pipe, connected to the negative pressure source and installed on said seal cover; a water inlet control valve, installed on the water inlet pipe; and a vacuum control valve, installed on the vacuum pipe; wherein said water absorbing material is adhered to the inner wall of said article storing bag or set in the article storing bag.

According to the further feature of the present invention, the article storing bag is transparent plastic bag.

According to the further feature of the present invention, the seal cover and container are made of transparent material.

Super absorbent polymer (SAP) is a functional polymer material. It has super absorbent functional and can absorb several hundreds or thousands times of its own weight, and can hold water very well. Once it absorb water and swell, the water will difficult to be separated even being forcing. So there are worldwide used in many fields, such as daily life, industrial and agriculture manufacture and building.

Super absorbent polymer (SAP) is a sort of polymer which contains hydrophilic group and crosslink structure, and firstly prepared by Fanta using starch graft polyacrylonitrile and then saponification. Sorted by raw materials, there are starch series(grafts, carboxy methylation, etc.), cellulose series(grafts, carboxy methylation, etc.), synthetic polymer series(polyacrylic acid series, polyvinyl alcohol series, polyoxyethylene series, etc.) and so on. Therein, compared with starch series and cellulose series, polyacrylic acid series Super absorbent polymer has lots of virtues, such as lower manufacturing cost, simple techniques, high production efficiency, Super absorbent capability, long shelf life, and so on, so it becomes currently research focus in the field. At present, polyacrylic acid series super absorbent polymer has taken 80% of super absorbent polymer production in the world.

Medical sewage treater consists of an article storing bag and water absorbing material. The said water absorbing material is super absorbent polymer, commonly using polyacrylic acid series super absorbent polymer which is lower manufacturing cost, super absorbent capability, long shelf life. Super absorbent polymer is adhered to the inner wall of the article storing bag or set in the article storing bag, then can obtain the invention. After Medical sewage flows into the article storing bag, the absorbent material will absorb water and swell. After that, medical sewage become colloid or solid and is expediently transported, which overcomes shortcomings in the existing technology during the medical sewage transportation. The said shortcomings are that medical sewage is not easy to pile up and prone to leaking which will lead to pollution. Besides, the invention can be used for collecting and disposing the deleterious sewage in daily life, and can make the transportation easy and safe.

### Brief Description of Drawings

The invention is herein described, by way of example only, with reference to the accompanying drawings. In the figures:
Fig.1 is a perspective view illustrating structure of medical sewage treater, in which inner wall adhered water absorbing material;
Fig.2 is a perspective view illustrating structure of medical sewage treater, in which an article storing bag contained water absorbing material;
Fig.3 is a perspective view illustrating structure of medical sewage treater with a seal cover;
Fig.4 is a perspective view illustrating structure of medical sewage treater with a seal cover and container; and
Fig.5 is a perspective view illustrating structure of medical sewage treater with a seal cover and water absorbing material contained bactericidal reagents.

In the above figures, 1 denotes the article storing bag; 2 denotes the water absorbing material, 3 denotes support pipe, 4 denotes container, 5 denotes bactericidal reagents or bactericidal materials, 11 denotes the mouth of the article storing bag, 31 denotes water inlet pipe, 32 denotes vacuum pipe, 311 denotes water inlet control valve, 312 denotes outlet-water of water inlet pipe, 321 denotes vacuum control valve, 322 denotes air inlet of the vacuum pipe.

### Best Mode for Carrying Out the Invention

### EXAMPLE 1: Medical sewage treater with inner wall adhered water absorbing material

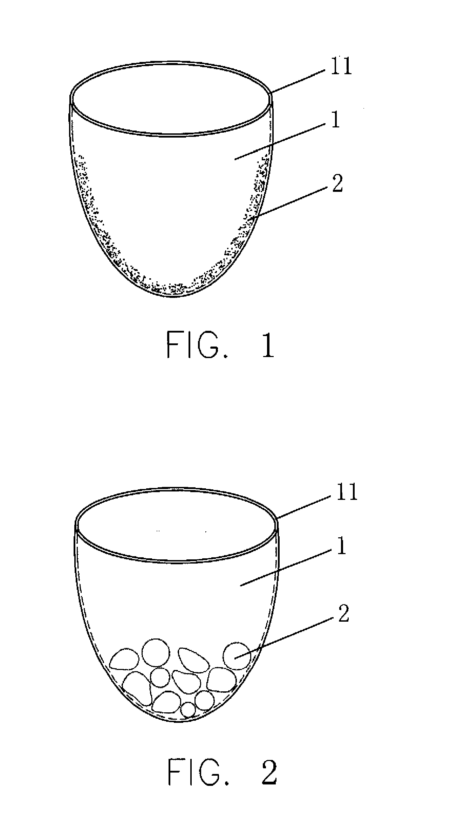

Reference is now made to FIG. 1, adopting high strength and transparent plastic film, cutting out and heat sealing, can prepare the article storing bag 1, and then spreading the water absorbing material on the inner wall of the article storing bag 1. After that, we can obtain Medical sewage treater with inner wall adhered water absorbing material. The water absorbing material 2, adopts Super absorbent polymer, it can be starch series(grafts, carboxy methylation, etc.), cellulose series(grafts, carboxy methylation, etc.), or synthetic polymer series (polyacrylic acid series, polyvinyl alcohol series, polyoxyethylene series, etc.). The water absorbing material 2 usually adopts polyacrylic acid series super absorbent polymer which is lower manufacturing cost, simple techniques, high production efficiency, super absorbent capability, long shelf life.

### EXAMPLE 2: Medical sewage treater with an article storing bag contained water absorbing material

The second embodiment of the medical sewage treater seen in FIG. 2 is generally similar to the first embodiment of FIG. 1 with the exception that in this example, the water absorbing material 2 is set in the article storing bag 1, and then can obtain the medical sewage treater with an article storing bag contained water absorbing material. The water absorbing material which is set in the article storing bag may be powder, particle, and rod or piece shape.

### EXAMPLE 3: Medical sewage treater with a seal cover

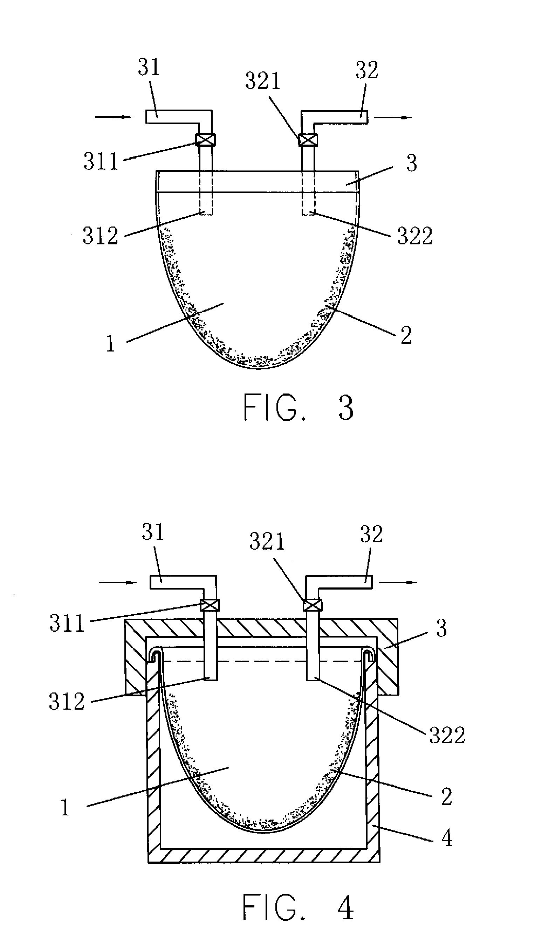

The third embodiment of the medical sewage treater seen in FIG. 3 is generally similar to the embodiments of FIG. 1 and 2 with the exception that in this example, a sealing cover 3 is installed on the mouth 11 of the article storing bag 1, and is connected to the mouth 11. Also, the inlet pipe 31and vacuum pipe 32 which is connected to the negative pressure source is installed on the sealing cover 3. There is an inlet water-control valve 311 on the inlet pipe 31 to open or close the inlet pipe, or to control the liquid flow in the inlet pipe. There is a vacuum control valve 312 on the vacuum pipe 32 to open or close the vacuum pipe, or to control the air flow in the vacuum pipe. The said water absorbing material 2 is adhered to the inner wall of the article storing bag 1 or set in the article storing bag 1. And thus the medical sewage treater with a sealing cover is made. The control valves can be replaced by caps (not shown in the figures) to open and close the inlet pipe and vacuum pipe.

To used, connect the vacuum pipe with the negative pressure source. Open the inlet water-control valve 311, and medical sewage can be sucked through inlet pipe 31. The water absorbing material absorbs the medical sewage sucked into the article storing bag 1, and expands. After that, medical sewage become colloid or solid of poor flowability, and can be easily transported. Thus the said invention can effectively overcome the disadvantages in the prior art, such as uneasily stacking, leakage and thereby pollution during the transportation of the medical sewage.

Besides, the invention can be used for collecting and disposing the deleterious sewage in daily life, as well as ensure easy and safe transportation of the sewage.

The product can protect environment, prevent and control pollution effectively under the situations such as field rescue, acute infectious disease treatment and sudden harmful liquid leakage.

### EXAMPLE 4: Medical sewage treater with a seal cover and container.

The fourth embodiment of the medical sewage treater seen in FIG. 4 is generally similar to the embodiments of FIG. 1 and 2 with the exception that in this example, a article storing bag 1 is placed into container 4. The container 4 is connected to the sealing cover 3. The water-outlet end of inlet pipe 312 and air-inlet end of the vacuum pipe 322 are placed within the article storing bag 1. The said water absorbing material is adhered to the inner wall of the article storing bag or set in the article storing bag. Thus, the medical sewage treater with a sealing cover and container is made.

Seal cover 3 and container 4 can be connected by concave-convex insertion, or screw , or other forms of mechanical or electromechanical matching. This type of medical sewage treater, can easily reused by changing the article storing bag 1 with water absorbing material 2 inside.

### EXAMPLE 5: Medical sewage treater with a seal cover and water absorbing material contained bactericidal reagents.

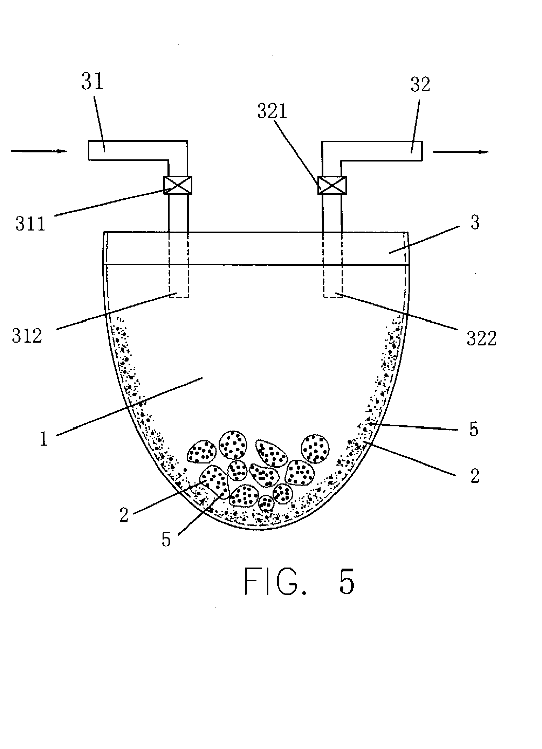

The fifth embodiment of the medical sewage treater seen in FIG. 5 is generally similar to the embodiments of FIG. 1 and 2 with the exception that in this example, the water absorbing material 2 contains bactericidal materials 5. When the medical sewage become colloid or solid of poor flowability, the bacteria in the medical sewage can be killed, this can further prevent the pollution and reduce the risk during the transportation.

It should be noted that the structure mentioned above in this invention can be replaced by any other structure sharing the same effects, and the examples introduced in this invention are not single structures that can be performed. Although the above examples are presented in this invention, it is to be understood by those who skilled in the art that numerous variations, improvements and substitutes may be effected without departing from the invention. Therefore, it should be define the range of protection according to the spirit and scope of the claims of this invention.

## Claims

1. A medical sewage treater comprising of an article storing bag (1) and water absorbing material (2), wherein said water absorbing material (2) is material which could make the treated medical sewage become a colloid or solid of poor flowability, and said water absorbing material (2) is adhered to the inner wall of said article storing bag (1) or set in the article storing bag (1).

2. The medical sewage treater according to claim 1, wherein said water absorbing material (2) is super absorbent polymer.

3. The medical sewage treater according to claim 2, wherein said super absorbent polymer is selected from a group of starch series, cellulose series or synthetic polymer series super absorbent polymer.

4. The medical sewage treater according to claim 2, wherein said super absorbent polymer is polyacrylic acid series super absorbent polymer.

5. The medical sewage treater according to claim 1, wherein said water absorbing material (2) is in the form of powder or particle.

6. The medical sewage treater according to claim 1, wherein said water absorbing material (2) contains bactericidal reagents or bactericidal materials (5).

7. The medical sewage treater according to claim 1, wherein medical sewage treater further comprising
a seal cover (3), connected to the mouth of the article storing bag (1);
a water inlet pipe (31);
a vacuum pipe (32), connected to the negative pressure source and installed on said seal cover;
a water inlet control valve (311), installed on the water inlet pipe (31); and
a vacuum control valve (312), installed on the vacuum pipe (32);
wherein said water absorbing material (2) is adhered to the inner wall of said article storing bag(1) or set in the article storing bag(1).

8. The medical sewage treater according to claim 7, wherein medical sewage treater further comprising a container(4); said article storing bag(1) is set in said container(4) and connect with the seal cover(3); water inlet control valve(311) on the water inlet pipe(31) and vacuum control valve(312) on the vacuum pipe are set in the article storing bag(1); wherein said water absorbing material(2) is adhered to the inner wall of said article storing bag(1) or set in the article storing bag(1).

9. The medical sewage treater according to claim 1, wherein said article storing bag (1) is transparent plastic bag.

10. The medical sewage treater according to claim 7, wherein said seal cover (3) and container (4) are made of transparent material.
